# EUROPÄISCHE PATENTSCHRIFT

(11) **EP 3 261 569 B1**
(45) Veröffentlichungstag und Bekanntmachung des Hinweises auf die Patenterteilung: **13.11.2019**
(21) Anmeldenummer: 16708584.4
(22) Anmeldetag: 30.01.2016
(51) Int. Cl.: A61B 1/00, A61B 17/00, A61B 17/32, A61B 18/00, A61B 18/08, A61B 18/14, A61J 15/00

(54) **ENDOSKOPISCHE SCHNEIDEINRICHTUNG MIT EINEM DURCH ÖFFNUNGEN IN EINEM TUBUS ZUR BILDUNG VON ZWEI DIATHERMIEMESSERN GEFÜHRTEN SCHNEIDDRAHT**
ENDOSCOPIC CUTTING DEVICE HAVING A CUTTING WIRE DIRECTED THROUGH OPENINGS IN A TUBE TO FORM TWO DIATHERMY BLADES
DISPOSITIF DE COUPE ENDOSCOPIQUE COMPORTANT UN FIL DE COUPE GUIDÉ À TRAVERS DES ORIFICES DANS UN TUBE POUR FORMER DEUX COUTEAUX DIATHERMIQUES

(30) Priorität: 23.02.2015 DE 102015102542
(43) Veröffentlichungstag der Anmeldung: 03.01.2018
(73) Patentinhaber: Medwork GmbH, 91315 Höchstadt/Aisch (DE)
(72) Erfinder: BREHM, Andreas, 91325 Adelsdorf (DE); STIRNWEISS, Matthias, 91475 Lonnerstadt (DE); KLYEISEN, Jörg, 91336 Heroldsbach (DE)
(74) Vertreter: Carstensen, Lars
(86) Internationale Anmeldenummer: PCT/DE2016/100038
(87) Internationale Veröffentlichungsnummer: WO 2016/134693

(56) Entgegenhaltungen:
- WO-A1-2012/160134
- DE-A1- 2 426 781
- DE-A1- 2 808 546
- DE-A1-102011 085 721
- US-A- 2 008 525

## Beschreibung

Die Erfindung betrifft eine endoskopische Schneideinrichtung zum Freischneiden einer mit einer Halteplatte versehenen Sonde, die im Rahmen einer perkutanen endoskopischen Gastrostomie in einer Magenwand angeordnet und von der Magenmukosa überwuchert ist, wobei zur Verstellung des an einem distalen Ende eines Tubus' vorgesehenen Schneidelements auf dieses eine Betätigungskraft eines proximal vorgesehenen Betätigungselements über ein Zugseil übertragbar ist, das in einem Lumen des Tubus' geführt ist, wobei die Schneideinrichtung einen mit dem Zugseil verbundenen elektrisch aufheizbaren Schneiddraht aufweist, der mit einem ersten Abstand zum distalen Ende am Tubus fixiert ist, von diesem aus außerhalb des Tubus' über einen distalen Abschnitt verläuft und über eine im Tubus vorgesehene erste Öffnung, die mit einem zweiten längeren Abstand zum distalen Ende des Tubus' vorgesehen ist, in das Lumen geführt ist, so dass sich beim Spannen des Zugseils der Abschnitt des Tubus' bogenförmig verformt und der Schneiddraht ein sehnenartig zwischen der ersten Öffnung und seiner Fixierung am Tubus verlaufendes erstes Diathermiemesser bildet.

Weiterhin wird eine Sonde für die perkutane endoskopische Gastrostomie beschrieben mit einem hohlzylindrischen Sondenkörper, der gemeinsam mit einer Halteplatte zur Anlage an einer Magenwand ausgebildet ist und nach einer Überwucherung durch die Magenmukosa mittels radialer Schnitte, die mit einer endoskopischen Schneideinrichtung durchgeführt werden, aus dieser herausgelöst werden, wobei zur Verstellung des an einem distalen Ende eines Tubus' vorgesehenen Schneidelements auf dieses eine Betätigungskraft eines proximal vorgesehenen Betätigungselements über ein Zugseil übertragbar ist, das in einem Lumen des Tubus' geführt ist, wobei die Schneideinrichtung einen mit dem Zugseil verbundenen elektrisch aufheizbaren Schneiddraht aufweist, der mit einem ersten Abstand zum distalen Ende am Tubus fixiert ist, von diesem aus außerhalb des Tubus' über einen distalen Abschnitt verläuft und über eine im Tubus vorgesehene erste Öffnung, die mit einem zweiten längeren Abstand zum distalen Ende des Tubus' vorgesehen ist, in das Lumen geführt ist, so dass sich beim Spannen des Zugseils der Abschnitt des Tubus' bogenförmig verformt und der Schneiddraht ein sehnenartig zwischen der ersten Öffnung und seiner Fixierung am Tubus verlaufendes erstes Diathermiemesser bildet.

Bei der perkutanen endoskopischen Gastrotomie wird dem Patienten endoskopisch ein künstlicher Zugang (PEG-Sonde) durch die Bauchdecke und die Magenwand in das Innere des Magens gelegt, um diesen mit Sondennahrung zu ernähren. Der Sondenkörper ist rohr- oder schlauchförmig ausgebildet und weist eine an der Magenwand vorgesehene Halteplatte auf, die die PEG-Sonde fixiert, um deren Bewegung nach außen zu verhindern. Diese Halteplatte liegt im Normalfall lose an der Magenwand an, und die PEG-Sonde bleibt während der gesamten über einen längeren Zeitraum erforderlichen Ernährung über diesen künstlichen Zugang funktionsfähig. Dafür ist es aber erforderlich, die Sonde etwa drei Tage nach der Anlage zu mobilisieren und diesen Vorgang regelmäßig in kurzen Abständen zu wiederholen.

Wird allerdings die regelmäßige Mobilisation versäumt oder durch eine dauerhafte Zugkraft auf die PEG-Sonde die Halteplatte entsprechend an die Magenwand gedrückt, so führt das zum Einwachsen der Halteplatte, da diese von der Magenmukosa überwuchert wird. Diese Komplikation, in deren Verlauf auch Entzündungen auftreten können, wird als Buried Bumper Syndrom bezeichnet. Letztendlich führt die Überwucherung der Halteplatte relativ häufig zu einer Sondendysfunktion, d.h., die wuchernde Magenmukosa bewirkt einen Verschluss der PEG-Sonde. In diesen Fällen ist es erforderlich, die von der Magenmukosa überwucherte PEG-Sonde zu entfernen und durch eine neue zu ersetzen.

Für die Entfernung der PEG-Sonde stehen unterschiedliche therapeutische Methoden zur Verfügung. Die PEG-Sonde kann durch einen chirurgischen Eingriff entfernt werden, der mit einer hohen Belastung für den ohnehin schon stark geschwächten Patienten verbunden ist. Bei der Laparotomie wird die Halteplatte durch chirurgische Schnitte, die von außen mittels eines über einen gelegten Zugang in das Mageninnere geführtes Lapraskop freigelegt. In Verbindung mit der Laparotomie kann auch die Pull-Methode angewandt werden, bei der die Sonde unter Zug nach außen entfernt wird.

Bei der Push-Methode wird zunächst ein Führungsdraht in die zunächst gekürzte PEG-Sonde eingeführt, woraufhin eine als Papillotom ausgebildete endoskopische Schneideinrichtung über den Führungsdraht im Bereich der Halteplatte positioniert wird. Mittels der endoskopischen Schneideinrichtung werden radiäre Einschnitte in die Überwucherungen der Halteplatte vorgenommen. Anschließend wird die PEG-Sonde mittels eines Bougies in das Mageninnere gedrückt. Die gekürzte PEG-Sonde kann anschließend über die Speiseröhre mittels endoskopischer Instrumente, wie beispielsweise eines Greifers oder einer Schlinge aus dem Magen entfernt werden.

Eine endoskopische Schneideinrichtung zum Freischneiden einer mit einer Halteplatte versehenen Sonde der im Oberbegriff des Patentanspruchs angegebenen Gattung, die im Rahmen einer perkutanen endoskopischen Gastrostomie in einer Magenwand angeordnet und von der Magenmukosa überwuchert ist, geht aus dem Dokument Cyrany J. et al; Cannulotoma introduced via a percutaneous endoscopic gastrostomy (PEG) tube - new technique for release of a buried bumper; Endoscopy 2012; 44; E422-E423 sowie aus dem Dokument Müller-Gerbes D. et al, Management bei Buried-Bumper-Syndrom: neue endoskopische minimalinvasive Push-Methode; Z.Gastroenterol 2009; 47; 1145-1148 als bekannt hervor. In beiden Dokumenten wird jeweils die zuvor erläuterte Push-Methode beschrieben, bei der als endoskopische Schneideinrichtung ein Papillotom verwendet wird, welches üblicher Weise für die Öffnung der Papilla Vateri vorgesehen ist, um die Entfernung von Gallensteinen zu erleichtern. Das Papillotom wird über die PEG-Sonde in den Magen eingeführt. Anschließend wird das Papillotom gespannt, so dass dessen distaler Endabschnitt einen gebogenen Verlauf einnimmt, der von einem Schneiddraht überspannt ist. Dieser Schneiddraht legt sich konvex an dem die Halteplatte überwuchernden Gewebe an und kann, verdreht in alle Richtungen, radiäre Schnitte ausführen. Die Halteplatte wird anschließend mit einem Bougie aus dem Mukosabett gelöst. Das Dokument DE 24 26 781 A1 beschreibt eine diathermische Schneidvorrichtung mit einem Führungsschlauch und mit einem Federdraht.

Es ist Aufgabe der vorliegenden Erfindung, eine Schneideinrichtung zur Behandlung des Buried Bumper Syndroms zu schaffen, die für die Push-Methode verwendbar ist und mit der eine Anzahl radiärer Schnitte durch die Überwucherungen bis an die innere Halteplatte der PEG-Sonde ausgeführt werden können.

Diese Aufgabe wird, ausgehend vom Oberbegriff des Patentanspruchs 1 in Verbindung mit den kennzeichnenden Merkmalen dieses unabhängigen Patentanspruchs gelöst. Die vom Patentanspruch 1 abhängigen Patentansprüche beinhalten erfindungsgemäße Weiterbildungen dieser Lösung.

Danach soll das distale Ende des Tubus zur Bildung einer Spitze unter einem Winkel zu dessen Querebene abgeschrägt sein, wobei die Spitze dem ersten Diathermiemesser zugewandt ist. Mit dem ersten Abstand zum Ende der Spitze ist eine zweite Öffnung im Tubus ausgebildet, über die der Schneiddraht in das Lumen eintritt und in diesem bis zur Spitze verläuft. Daran anschließend ist der Schneiddraht zur Bildung eines zweiten Diathermiemessers außen um die Spitze herumgeführt und, entlang einer Außenmantelfläche des Tubus verlaufend, über die zweite Öffnung in den Tubus zurückgeführt. Der Tubus besteht zumindest im Bereich seines zwischen den beiden Öffnungen liegenden Abschnitts aus einem biegeweichen, elastisch verformbaren Kunststoff.

Der elektrisch aufheizbare Schneiddraht verläuft somit nicht nur zwischen den beiden im Tubus vorgesehenen Öffnungen, sondern er tritt außerdem am distalen Ende des Tubus' aus dem Lumen aus, ist um die Spitze herumgeführt und verläuft außen am Tubus entlang in dessen axialer Richtung, um über die im distalen Endabschnitt vorgesehene Öffnung wieder in das Lumen einzutreten. Weiterhin ist die Spitze, die ein definiertes Eintauchen des von dieser ausgehenden Schneidedrahts in die Überwucherung und somit eine präzise Führung der Schneideinrichtung ermöglicht, als Abschrägung der Stirnfläche gestaltet. Dieser weitere wirksame Abschnitt des Schneiddrahtes dient somit als zweites Diathermiemesser. Da der zwischen den beiden Öffnungen liegende Abschnitt des Tubus' beim Schneidvorgang zu einem bogenförmigen Verlauf verformt wird, bei welchem die Tubusabschnitte um 180° zueinander gebogen sind, ist von besonderer Bedeutung, dass der Tubus zumindest in diesem Bereich seines zwischen den beiden Öffnungen liegenden Abschnitts aus einem biegeweichen, elastisch verformbaren Kunststoff besteht. Wie bereits dargelegt, werden bei dem endoskopischen Freischneiden der Halteplatte mehrere radiäre Schnitte ausgeführt, so dass der Abschnitt mehrfach extrem verformt werden und anschließend zum Verschwenken der Schneideinrichtung wieder in eine Stellung mit geringer Abwinkelung zurückgelangen muss.

Demgegenüber soll die Schneideinrichtung für die Push-Methode nach den Druckschriften Cyrany J. et al; Cannulotoma introduced via a percutaneous endoscopic gastrostomy (PEG) tube - new technique for release of a buried bumper; Endoscopy 2012; 44; E422-E423 sowie Müller-Gerbes D. et al, Management bei Buried-Bumper-Syndrom: neue endoskopische minimalinvasive Push-Methode; 2009; 47; 1145-1148 als übliches Papillotom ausgebildet sein. Bei diesem ist keine durch eine Abschrägung hergestellte distale Spitze vorgesehen. Weiterhin ist ein distales Ende des Schneiddrahtes am Tubus fixiert und somit nicht vom distalen Ende des Tubus, in axialer Richtung verlaufend, an der Außenmantelfläche des Tubus entlanggeführt. Daher kann ein vom distalen Ende der PEG-Sonde bis zur Halteplatte reichender Ansatz nicht umschnitten werden. Der distale Endabschnitt des Tubus wird nur in geringem Maße abgewinkelt, so dass dieser auch relativ steif ausgebildet sein kann.

Zur Führung des von der Spitze aus in axialer Richtung verlaufenden Schneiddrahtes kann der Schlauch in seinem Querschnitt eine Längsrille aufweisen. Weiterhin besteht die Möglichkeit, den Tubus beidseitig dieser Längsrille mit Abflachungen zu versehen, wobei die Abflachungen zueinander unter einem spitzen Winkel verlaufen. Daraus ergibt sich in diesem Schneidbereich eine Keilform, die ermöglicht, bereits getrenntes Gewebe bei Seite zu drücken. Damit wird Platz geschaffen für ein definiertes und sauberes Eintauchen des Schneidedrahts ins Zielgewebe. Der Tubus ist vorzugsweise aus einem Fluorpolymer, aus Polyamid oder Polyolefin hergestellt. Der Tubus kann zweilumig ausgebildet sein, wobei innerhalb des einen Lumens die Litze des Schneiddrahtes und, wie nachfolgend noch angegeben, ein PEEK-Schlauch und PEEK-Hülsen verlaufen. Über das zweite Lumen kann die Schneideinrichtung bei ihrem Einführen in die PEG-Sonde an einem Führungsdraht geführt sein. Außerdem kann durch das zweite Lumen eine Spülflüssigkeit zum distalen Ende der Schneideinrichtung geleitet werden.

In Weiterbildung der Erfindung soll sich ein distaler Endabschnitt des Schneiddrahtes innerhalb des Lumen über mindestens 50% des zwischen den beiden Öffnungen liegenden Bereichs erstrecken. Der im Lumen in proximaler Richtung zurückgeführte Schneiddraht unterstützt die Rückstellung des Tubus, wenn das Zugseil über das Betätigungselement entspannt wird, und wirkt somit wie ein Federelement. Dabei kann sich der distale Endabschnitt innerhalb des Lumens bis zur ersten Öffnung erstrecken.

Außerdem soll das distale Ende des Schneiddrahtes um etwa 180° umgebogen sein, um dieses an der Mantelfläche des Lumens zu fixieren. Das entsprechende Ende des Schneiddrahtes ist mit einem Endabstand von etwa 3mm um ca. 180° umgebogen, so dass dieses Ende die Wirkung eines im Lumen angreifenden Widerhakens aufweist. Durch die entsprechende Schneiddrahtführung und -befestigung werden keine weiteren Komponenten sowie Füge- und Befestigungsprozesse benötigt.

Weiterhin ist vorgesehen, dass angrenzend an die Öffnungen und außerhalb des zwischen den Öffnungen liegenden Bereiches PEEK-Hülsen in das Lumen eingesetzt sind. Dabei kann sich eine erste PEEK-Hülse innerhalb des Lumens von der Spitze bis zur zweiten Öffnung erstrecken. Bevor der Schneiddraht durch die erste oder zweite Öffnung aus dem Lumen austritt, ist das Lumen jeweils durch eine PEEK-Hülse verstärkt, so dass der Tubus in diesem Bereich nicht einreißen kann. Die beiden PEEK-Hülsen sind innerhalb des Lumens verklemmt. Mittels der ersten PEEK-Hülse ist der Tubus an seinem distalen Endabschnitt, also zwischen der Spitze und der zweiten Öffnung verstärkt, so dass der Tubus im Umlenkbereich des Schneiddrahtes an der Spitze nicht einreißen kann und der distale Endabschnitt des Tubus zum Eindringen in die Überwucherungen steif ausgebildet ist. Polyetheretherketon (abgekürzt PEEK) ist ein hochtemperaturbeständiger thermoplastischer Kunststoff und gehört zur Stoffgruppe der Polyaryletherketone. Die Hülsen können auch aus einem anderen einreißfesten Kunststoff oder aus Metall hergestellt sein.

Um den Tubus partiell biege- und rotationssteif zu gestalten, ist ein innerhalb des Lumens verlaufender PEEK-Schlauch vorgesehen, der mit seinem proximalen Ende in einer Aufnahme eines Führungsschafts des Betätigungselements fixiert ist und distal an einer Stirnseite einer zweiten PEEK-Hülse anliegt, wobei sich die zweite PEEK-Hülse bis zur ersten Öffnung erstreckt. Der PEEK-Schlauch ist innerhalb des Lumens, von einem Führungsschaft des Betätigungselements bis zur zweiten PEEK-Hülse verlaufend, angeordnet und dient zur Erhöhung der Biegefestigkeit und Torsionssteifigkeit des Tubus. Flexibel und relativ weich soll nur der zwischen den beiden Öffnungen liegende Bereich des Tubus sein, der sich beim Spannen des Zugseils bogenförmig krümmt. Aufgrund der Torsionssteifigkeit können Drehbewegungen am Betätigungselement verzögerungsfrei auf die Schneideinrichtung übertragen werden.

In weiterer Ausgestaltung der Erfindung soll das Betätigungselement einen zumindest mittelbar mit dem Tubus verbundenen Führungsschaft und ein am Zugseil angreifendes Schiebeelement aufweisen, zwischen denen ein Element zum Auslösen eines haptischen Signals vorgesehen ist, welches ausgelöst wird, wenn die Schneideinrichtung eine ihrer Schneidstellungen erreicht hat. Aufgrund dieses haptischen Signals kann der Chirurg erkennen, ob sich die Schneideinrichtung in einer ihrer Schneidstellungen befindet. Das Signal kann beispielsweise dann ausgelöst werden, wenn sich die Spitze im Bereich eines Randes der Halteplatte befindet, da dann der Schneiddraht bestromt werden kann, oder bei maximaler Krümmung des Tubus an einen Außenumfang einer Nabe der Halteplatte bewegt wird. Das Element zum Auslösen des haptischen Signals kann als Rastnut an einer Außenmantelfläche des Schiebeelements und als im Schiebeelement radial zu der Rastnut verlaufende federbelastete Kugel ausgebildet sein.

Schließlich soll die Sonde für die perkutane endoskopische Gastrostomie mit einem hohlzylindrischen Sondenkörper versehen sein, der gemeinsam mit einer Halteplatte zur Anlage an einer Magenwand ausgebildet ist und nach einer Überwucherung durch die Magenmukosa mittels radialer Schnitte, die mit einer endoskopischen Schneideinrichtung durchgeführt werden, aus dieser herausgelöst werden. Dabei ist zur Verstellung der an einem distalen Ende eines Tubus vorgesehenen Schneideinrichtung auf diese eine Betätigungskraft eines proximal vorgesehenen Betätigungselements über ein Zugseil übertragbar. Das Zugseil ist in einem Lumen eines Tubus geführt, wobei die Schneideinrichtung einen mit dem Zugseil verbundenen elektrisch aufheizbaren Schneiddraht aufweist, der mit einem ersten Abstand zum distalen Ende am Tubus fixiert ist, von diesem aus außerhalb des Tubus über einen distalen Abschnitt verläuft und über eine im Tubus vorgesehene erste Öffnung, die mit einem zweiten längeren Abstand zum distalen Ende des Tubus vorgesehen ist, in das Lumen geführt ist. Daher verformt sich beim Spannen des Zugseils der Abschnitt des Tubus bogenförmig, und der Schneiddraht bildet ein sehnenartig zwischen der ersten Öffnung und seiner Fixierung am Tubus verlaufendes erstes Diathermiemesser.

Dabei ist ein distales Ende des Sondenkörpers, das einen axialen Überstand über die Halteplatte aufweist, hinsichtlich seiner axialen und radialen Abmessungen an die Lage des Schneiddrahtes und die Lage eines als Spitze ausgebildeten distalen Endes des Tubus angepasst, die diese während eines Schneidvorganges einnehmen. Die auf die Abmessungen der Halteplatte und der Nabe der Sonde abgestimmte Schneiddrahtlänge und der Abstand der Spitze zur Mitte der bogenförmigen Verformung des Tubus bewirken, dass die Schneideinrichtung von oben direkt am Rand der Nabe angreift. Durch weiteres Anspannen fährt die vom zweiten Diathermiemesser umhüllte Spitze langsam in Richtung des Zentrums der PEG-Sonde. In voll angespannter Stellung des Tubus ist die Nabe der PEG-Sonde komplett, also sowohl in axialer als auch in radialer Richtung vom Schneiddraht umfasst, so dass ein Schnitt ausgeführt wurde.

Die Bezugnahme der Patentansprüche auf die Zeichnung durch die Verwendung von Bezugszeichen soll den Schutzumfang der Patentansprüche nicht beschränken.

Zur weiteren Erläuterung der Erfindung wird auf die Zeichnungen verwiesen, in denen ein Ausführungsbeispiel vereinfacht dargestellt ist. Es zeigen:
- Figur 1: eine Behandlung eines Buried Bumper Syndroms mittels einer erfindungsgemäß ausgebildeten endoskopischen Schneideinrichtung,
- Figur 2: einen Längsschnitt der gemäß Figur 1 verwendeten endoskopischen Schneideinrichtung,
- Figur 3: einen vergrößerten Ausschnitt gemäß III aus der Darstellung nach Figur 2, der den Bereich einer in einem Tubus vorgesehenen ersten Öffnung zeigt, über die ein Schneiddraht aus einem Lumen austritt,
- Figur 4: einen vergrößerten Ausschnitt gemäß IV aus der Darstellung nach Figur 2, der einen Führungsschaft eines Betätigungselements zeigt, an dem sowohl der Tubus als auch ein PEEK-Schlauch fixiert sind,
- Figur 5: im Längsschnitt eine Teilansicht der Schneideinrichtung im Bereich ihres distalen Endes,
- Figur 6: im Teillängsschnitt ein Betätigungselement der Schneideinrichtung,
- Figur 7: einen vergrößerten Ausschnitt gemäß VII aus der Darstellung nach Figur 6, der eine Anordnung zur Erzeugung eines haptischen Signals zeigt,
- Figur 8: eine alternative Ausführungsform des distalen Endabschnittes, wobei der Tubus in diesem Bereich mit Abflachungen versehen ist und somit keilförmig gestaltet ist,
- Figur 9: einen Längsschnitt des Schneidbereichs der Schneideinrichtung,
- Figur 10: einen Längsschnitt durch eine PEG-Sonde mit einer in dieser angeordneten Schneideinrichtung, wobei diese sich im gestreckten Zustand befindet,
- Figur 11: den Zustand der Schneideinrichtung, in welchem der Tubus soweit gebogen ist, dass der Schneiddraht an der ersten Öffnung um ca. 45° abgewinkelt ist,
- Figur 12: den Zustand der Schneideinrichtung, in welchem der Tubus soweit gebogen ist, dass dessen distaler Endabschnitt unter einem Winkel von ca. 180° zum proximalen Abschnitt verläuft, wobei eine Spitze des Tubus am Rand einer Halteplatte angreift, und
- Figur 13: den Zustand der Schneideinrichtung, in welchem der Tubus soweit gebogen ist, dass dessen distaler Endabschnitt unter einem Winkel von ca. 180° zum proximalen Abschnitt verläuft, wobei die Spitze bis an eine an der Halteplatte vorgesehene Nabe geführt worden ist.

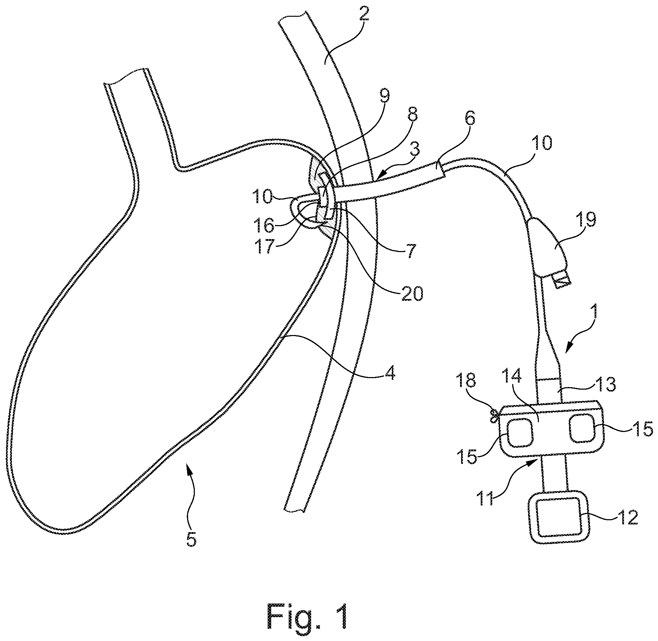

In der Figur 1 ist mit 1 eine endoskopische Schneideinrichtung bezeichnet, die zur Behandlung eines Buried Bumper Syndroms vorgesehen ist. In eine Bauchdecke 2 eines Patienten ist eine PEG-Sonde 3 zu dessen Ernährung eingesetzt, die eine Magenwand 4 eines Magens 5 durchdringt. Die PEG-Sonde 3 besteht aus einem hohlzylindrischen, vorzugsweise als Schlauch ausgebildeten Sondenkörper 6 und einer mit diesem verbundenen inneren Halteplatte 7, die im Inneren des Magens 5 an der Magenwand 4 anliegt, wodurch die PEG-Sonde 3 fixiert ist. Der Sondenkörper 6 überragt die Halteplatte 7, wodurch eine Nabe 8 gebildet wird. Wie weiterhin aus der Figur 1 hervorgeht, ist die Halteplatte 7 an ihrer dem Mageninneren zugewandten Oberfläche von Magenmukosa 9 überwuchert bzw. in die Magenwand 4 eingewachsen. Das bedeutet, dass im Bereich dieser Überwucherungen Entzündungsherde entstehen können und erhebliche Probleme bestehen, die PEG-Sonde aus der Magenwand bzw. der Bauchdecke 2 zu entfernen.

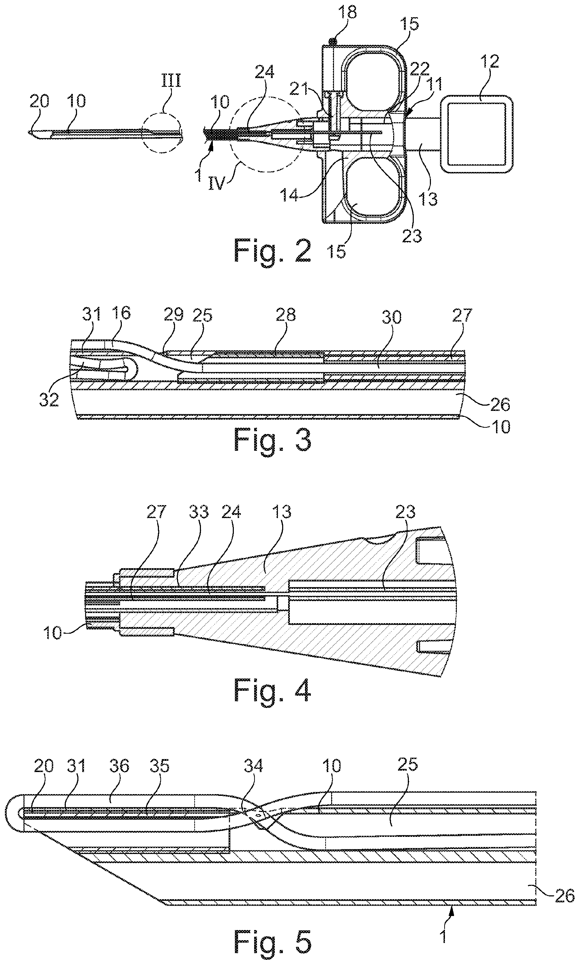

Zum Freischneiden der Halteplatte 7 ist die endoskopische Schneideinrichtung 1 über die PEG-Sonde 3 in das Innere des Magens 5 geführt. Diese weist einen aus einem biegeweichen, flexiblen Kunststoff hergestellten Tubus 10 auf, an dessen proximalen Ende ein Betätigungselement 11 vorgesehen ist. Über dieses Betätigungselement 11, das einen mit einem Daumenring 12 verbundenen Führungsschaft 13 und ein Schiebeelement 14 mit zwei daran ausgebildeten Fingerringen 15 aufweist, kann ein distaler Endabschnitt des Tubus 10 derart bogenförmig verspannt werden, dass ein diesen Bogen überspannender Schneiddraht 16 als erstes Diathermiemesser 17, das über einen Hochfrequenzanschluss 18 bestromt und somit erhitzt wird. Außerdem ist am Tubus 10 eine Schleuse 19 vorgesehen, über die ein Führungsdraht und/oder eine Spülflüssigkeit zugeführt werden können, die über ein im Zusammenhang mit den Figuren 2, 5 und 9 erläutertes Lumen bis an das distale Ende des Tubus' 10 verlaufen bzw. geleitet werden. An seinem distalen Ende ist der Tubus 10 mit einer Spitze 20 versehen, die in der bogenförmig gekrümmten Stellung dieses Endbereichs, die in der Figur 1 dargestellt ist, in die die Halteplatte 7 überwuchernde Magenmukosa 9 einsticht.

In der Figur 2 ist die endoskopische Schneideinrichtung 1 in einem gestreckten Zustand dargestellt, in welchem sie sich beispielsweise befindet, bevor deren distales Ende in eine PEG-Sonde 3 nach Figur 1 eingeführt wird. Die bereits im Zusammenhang mit der Figur 1 erläuterten Details der Schneideinrichtung 1, die auch in der Figur 2 gezeigt werden, sind mit den gleichen Bezugsziffern versehen. Darüber hinaus geht aus der Figur 2 hervor, dass von dem Schiebeelement 14 aus ein Mitnehmer 21 radial nach innen ragt und in einem Längsschlitz 22 des Führungsschafts 13 geführt ist. An diesem Mitnehmer ist eine Zugstange 23 befestigt, an die sich im weiteren Verlauf ein Zugseil 24 anschließt.

Ein erster vergrößerter Ausschnitt III aus der Figur 2 ist in der Figur 3 dargestellt. Dieser zeigt einen Teilabschnitt des Tubus' 10 mit einem in diesem verlaufenden ersten Lumen 25 sowie einem zweiten Lumen 26. In dem ersten Lumen 25 ist proximal ein PEEK-Schlauch 27 angeordnet, der, wie nachfolgend noch in den weiteren Figuren gezeigt wird, bis zum Führungsschaft 13 des Betätigungselements 11 reicht. Mittels des PEEK-Schlauches 27 wird der insgesamt biegeweiche und flexible Tubus 10 partiell versteift, so dass sich über diesen Schub- und Drehbewegungen auf einen distalen Endabschnitt des Tubus' 10, die von dem Betätigungselement 11 ausgehen, übertragen lassen.

In distaler Richtung schließt sich innerhalb des ersten Lumens 25 an den PEEK-Schlauch 27 eine zweite PEEK-Hülse 28 an, die distal vor einer ersten Öffnung 29 schräg endet. Dabei ist das schräge Ende dieser zweiten PEEK-Hülse 28 derart ausgerichtet, dass es auf die erste Öffnung 29 zuläuft. Innerhalb des PEEK-Schlauches 27 und der zweiten PEEK-Hülse 28 verläuft die als Schneiddraht 16 dienende Litze 30, die proximal mit dem Zugseil 24 verbunden ist. Der Schneiddraht 16 tritt über die erste Öffnung 29 aus der PEEK-Hülse 28 bzw. aus dem ersten Lumen 25 aus und verläuft entlang einer Außenmantelfläche 31 des Tubus' 10. Der weitere Verlauf des Schneiddrahtes 16, von welchem in der Figur 3 ein um 180° umgebogenes Ende 32 gezeigt wird, kann einigen der nachfolgend erläuterten Figuren entnommen werden. Aus der Figur 3 geht hervor, dass das Ende 32 bis an die erste Öffnung 29 reicht und derart ausgebildet ist, dass es sich unter Zugkraft innerhalb des ersten Lumens 25 festkrallt.

Ein zweiter vergrößerter Ausschnitt IV aus der Figur 2, der die Verbindung des Führungsschafts 13 mit dem Tubus 10 sowie mit dem PEEK-Schlauch 27 zeigt, ist in der Figur 4 dargestellt. Daraus geht hervor, dass der im ersten Lumen 25 geführte PEEK-Schlauch 27 in einer Aufnahme 33 des Führungsschafts 13 fixiert ist. Das innerhalb des PEEK-Schlauchs 27 verlaufende Zugseil 24 ist in der Zugstange 23 befestigt.

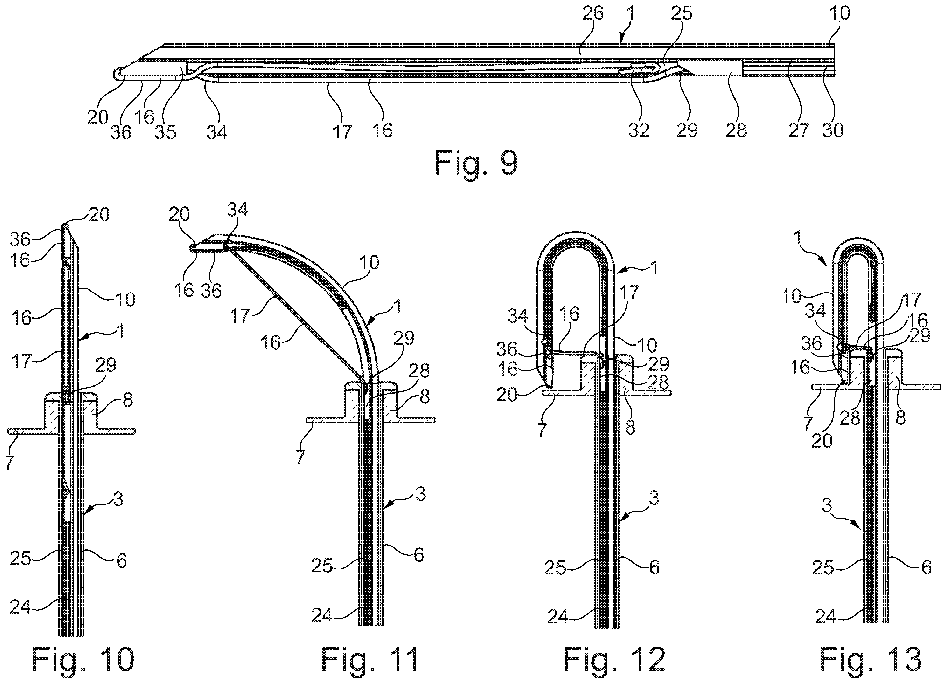

In der Figur 5 ist ein distales Ende der Schneideinrichtung 1 mit der Spitze 20 dargestellt, welche dadurch hergestellt wird, dass der Tubus 10 unter einem Winkel zur Querebene abgetrennt wird. Beabstandet zu dieser Spitze 20 ist im Tubus 10 eine zweite Öffnung 34 vorgesehen, die in das erste Lumen 25 mündet. Ausgehend vom distalen Ende des Tubus' 10 befindet sich in dem Lumen 25 eine erste PEEK-Hülse 35, die proximal bis zur zweiten Öffnung 34 reicht. Der gesamte Verlauf des Schneiddrahtes 16 im Bereich der beiden Öffnungen 29 und 34 sowie der Spitze kann im Übrigen der Figur 9 entnommen werden. Danach soll der an der Außenmantelfläche 31 entlanglaufende Schneiddraht 16 über die zweite Öffnung 34 in das erste Lumen 25 geführt werden, in der er bis zur Spitze 20 verläuft.

An der Spitze 20 wird der Schneiddraht 30 um 180° umgelenkt und verläuft anschließend unter Bildung eines zweiten Diathermiemessers 36 wiederum an der Außenmantelfläche 31 bis zur zweiten Öffnung 34, um an dieser wiederum in das erste Lumen 25 einzutreten. Dieser in proximaler Richtung verlaufende Endabschnitt des Schneiddrahtes 16 ist, wie aus den Figuren 3 und 9 hervorgeht, mit dem um 180° umgebogenen Ende 32 versehen. Die Figur 9 zeigt, dass sich der Endabschnitt über den zwischen den beiden Öffnungen 29 und 34 liegenden Bereich erstreckt.

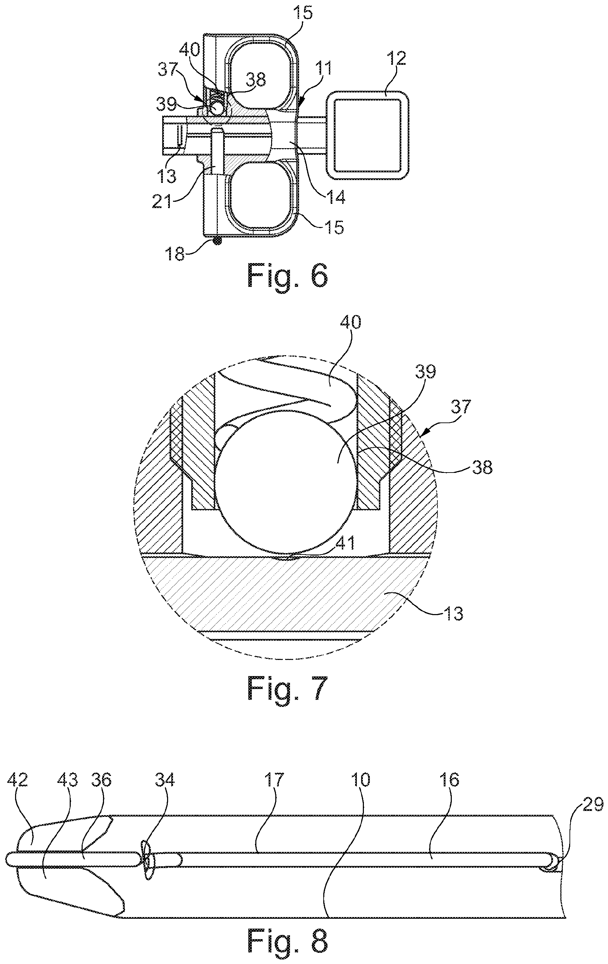

Die Figur 6 zeigt das Betätigungselement 11, welches in diesem Fall mit einem Element 37 zur Erzeugung eines haptischen Signals versehen ist. Zu diesem Zweck ist in einer radial verlaufenden Bohrung 38 des Schiebeelements 14 eine Kugel 39 angeordnet. Ein vergrößerter Ausschnitt VII aus der Figur 6, der die Ausbildung des Elements 37 zur Erzeugung eines haptischen Signals zeigt, ist in der Figur 7 dargestellt. Zur Verdeutlichung der Funktion dieses Elements 37 ist die von einer Druckfeder 40 beaufschlagte Kugel 39 in einer Stellung gezeigt, in der sie von dem Führungsschaft 13 abgehoben ist, so dass eine auf dem Führungsschaft 13 angeordnete Rastausnehmung 41 sichtbar ist. In diese Rastausnehmung 41 rastet die Kugel 39 ein, wenn die Schneideinrichtung 1 sich in einer Schneidstellung befindet, was der behandelnde Chirurg am Betätigungselement 11 spürt.

In der Figur 8 ist ein distaler Endabschnitt eines weiteren Ausgestaltungsbeispiels der endoskopischen Schneideinrichtung 1 dargestellt, bei dem der Tubus 10 an seinem die Spitze 20 aufweisenden Endabschnitt zumindest teilweise mit Abschrägungen 42 und 43 versehen ist, so dass sich in diesem Bereich eine keilförmige Ausbildung des Tubus' 10 ergibt. Dadurch wird während des Schneidvorgangs des zweiten Diathermiemessers 36 die voneinander getrennte Magenmukosa auseinander gedrückt.

In den Figuren 10 bis 13 ist die in die PEG-Sonde 3 eingeschobene endoskopische Schneideinrichtung in ihren unterschiedlichen Stellungen dargestellt. Die PEG-Sonde besteht, wie bereits im Zusammenhang mit der Figur 1 erläutert, aus der Halteplatte 7, der sich von dieser in das Mageninnere erstreckenden Nabe 8 und dem als Schlauch ausgebildeten Sondenkörper 6. Die Figur 10 zeigt die Schneideinrichtung 1 in ihrem gestreckten Zustand nachdem diese in die PEG-Sonde 3 eingeführt wurde. Nach der Figur 11 verläuft der Schneiddraht 16 aufgrund einer Abwinklung des Tubus' 10 um ca. 90° unter einem Winkel von 45°. Dadurch richtet sich die Schneideinrichtung 1 mittels des am Ende der Nabe 8 anliegenden Schneiddrahtes in axialer Richtung aus.

Gemäß der Figur 12 ist das Zugseil derart angespannt, dass der distale Endabschnitt des Tubus' 10 um 180° gebogen ist und somit parallel zum übrigen Teil des Tubus' 10 verläuft. Dadurch wird die Spitze 20 mit dem um sie herumgeführten Schneiddraht 16 am Rand der Halteplatte 7 angesetzt. Diese Stellung wird dem Chirurgen, wie im Zusammenhang mit den Figuren 6 und 7 erläutert, signalisiert und er schaltet den Hochfrequenzstrom ein. Anschließend spannt er das Zugseil weiter an, und es wird über die Halteplatte 7 ein radiärer Schnitt ausgeführt, bei dem sowohl das erste Diathermiemesser 17 als auch das zweite Diathermiemesser 36 wirksam sind.

Nach einem Entspannen des Zugseils und einem jeweiligen Verdrehen der Schneideinrichtung 1 werden die Schnitte mehrfach wiederholt. Da der Tubus 10 in seinem Biegebereich biegeweich ausgeführt ist und aufgrund des innerhalb des Lumens 25 zurückgeführten Schneiddrahtes 16 flexibel ist, kann zum einen der Tubus 10 sehr stark abgewinkelt werden und gelangt zum anderen wieder in seine vorangegangene Position zurück. Die PEEK-Hülsen 28 und 35 verhindern dabei, dass der Tubus 10 im Bereich der Öffnungen 29 und 34 einreißt.

### Bezugszeichenliste

1 endoskopische Schneideinrichtung
2 Bauchdecke
3 PEG-Sonde
4 Magenwand
5 Magen
6 Sondenkörper
7 Halteplatte von 3
8 Nabe von 3
9 Magenmukosa
10 Tubus
11 Betätigungselement
12 Daumenring von 11
13 Führungsschaft von 11
14 Schiebeelement von 11
15 Fingerring
16 Schneidedraht
17 erstes Diathermiemesser
18 Hochfrequenzanschluss
19 Schleuse
20 Spitze
21 Mitnehmer
22 Längsschlitz
23 Zugstange
24 Zugseil
25 erstes Lumen
26 zweites Lumen
27 PEEK-Schlauch
28 zweite PEEK-Hülse
29 erste Öffnung
30 Litze
31 Außenmantelfläche von 10
32 Ende von 16
33 Aufnahme in 13
34 zweite Öffnung
35 erste PEEK-Hülse
36 zweites Diathermiemesser
37 Element
38 Bohrung
39 Kugel
40 Druckfeder
41 Rastausnehmung

## Patentansprüche

1. Endoskopische Schneideinrichtung (1) für eine Verwendung beim Freischneiden einer mit einer Halteplatte (7) versehenen, im Rahmen einer perkutanen endoskopischen Gastrostomie in einer Magenwand (4) angeordneten und von der Magenmukosa (9) überwucherten Sonde (3), wobei die Schneideinrichtung (1) ein an einem distalen Ende eines Tubus (10) vorgesehenes Schneidelement aufweist, zu dessen Verstellung auf dieses eine Betätigungskraft eines proximal vorgesehenen Betätigungselements (11) über ein Zugseil (24) übertragbar ist, das in einem Lumen (25) des Tubus (10) geführt ist, wobei die Schneideinrichtung (1) einen mit dem Zugseil (24) verbundenen elektrisch aufheizbaren Schneiddraht (16) aufweist, der mit einem ersten Abstand zum distalen Ende am Tubus (10) fixiert ist, von diesem aus außerhalb des Tubus (10) über einen distalen Abschnitt verläuft und über eine im Tubus (10) vorgesehene erste Öffnung (29), die mit einem zweiten längeren Abstand zum distalen Ende des Tubus (10) vorgesehen ist, in das Lumen (25) geführt ist, so dass sich beim Spannen des Zugseils (24) der Abschnitt des Tubus (10) bogenförmig verformt und der Schneiddraht (16) ein sehnenartig zwischen der ersten Öffnung (29) und seiner Fixierung am Tubus (10) verlaufendes erstes Diathermiemesser (17) bildet, wobei mit dem ersten Abstand zum Ende der Spitze (20) eine zweite Öffnung (34) im Tubus (10) ausgebildet ist, über die der Schneiddraht (16) in das Lumen (25) eintritt und wobei der Tubus (10) zumindest im Bereich seines zwischen den beiden Öffnungen (29 und 34) liegenden Abschnitts aus einem biegeweichen, elastisch verformbaren Kunststoff besteht, **dadurch gekennzeichnet, dass** das distale Ende des Tubus (10) zur Bildung einer Spitze (20) unter einem Winkel zu dessen Querebene abgeschrägt ist, die dem ersten Diathermiemesser (17) zugewandt ist, dass der Schneiddraht (16) im Lumen (25) bis zur Spitze (20) verläuft und dass der Schneiddraht (16) zur Bildung eines zweiten Diathermiemessers (36) außen um die Spitze (20) herumgeführt ist und, entlang einer Außenmantelfläche (31) des Tubus (10) verlaufend, über die zweite Öffnung (34) in den Tubus (10) eintritt.

2. Endoskopische Schneideinrichtung nach Patentanspruch 1, **dadurch gekennzeichnet, dass** sich ein distaler Endabschnitt des Schneiddrahtes (16) innerhalb des Lumens (25) über mindestens 50% des zwischen den beiden Öffnungen (29 und 34) liegenden Bereichs erstreckt.

3. Endoskopische Schneideinrichtung nach Patentanspruch 2, **dadurch gekennzeichnet, dass** sich der distale Endabschnitt innerhalb des Lumens (25) bis zur ersten Öffnung (29) erstreckt.

4. Endoskopische Schneideinrichtung nach Patentanspruch 1, **dadurch gekennzeichnet, dass** der außen um die Spitze (20) herumgeführte und entlang der Außenmantelfläche (31) des Tubus (10) verlaufende Schneiddraht (16), der über die zweite Öffnung (34) in das Lumen (25) des Tubus (10) eintritt, an seinem distalen Ende um etwa 180° umgebogen und mit diesem an einer Innenmantelfläche des Lumens (25) fixiert ist.

5. Endoskopische Schneideinrichtung nach Patentanspruch 2, **dadurch gekennzeichnet, dass** angrenzend an die Öffnungen (29 und 34) und außerhalb des zwischen den Öffnungen (29 und 34) liegenden Bereiches PEEK-Hülsen (28 und 35) in den Tubus (10), vorzugsweise in das Lumen (25), eingesetzt sind.

6. Endoskopische Schneideinrichtung nach Patentanspruch 5, **dadurch gekennzeichnet, dass** sich eine erste PEEK-Hülse (35) innerhalb des Lumens (25) von der Spitze (20) bis zur zweiten Öffnung (34) erstreckt.

7. Endoskopische Schneideinrichtung nach Patentanspruch 5, **dadurch gekennzeichnet, dass** ein innerhalb des im Tubus (10) ausgebildeten Lumens (25) verlaufender PEEK-Schlauch (27) mit seinem proximalen Ende in einer Aufnahme (33) eines Führungsschafts (13) des Betätigungselements (11) fixiert ist und distal an einer Stirnseite einer zweiten PEEK-Hülse (28) anliegt, die sich bis zur ersten Öffnung (29) erstreckt.

8. Endoskopische Schneideinrichtung nach Patentanspruch 1, **dadurch gekennzeichnet, dass** das Betätigungselement (11) einen zumindest mittelbar mit dem Tubus (10) verbundenen Führungsschaft (13) und ein am Zugseil (24) angreifendes Schiebeelement (14) aufweist, zwischen denen ein Element (37) zum Auslösen eines haptischen Signals vorgesehen ist, welches ausgelöst wird, wenn die Schneideinrichtung (1) eine ihrer Schneidstellungen erreicht hat.

9. Endoskopische Schneideinrichtung nach Patentanspruch 8, **dadurch gekennzeichnet, dass** das Element (37) zum Auslösen des haptischen Signals als Rastausnehmung (41) an einer Außenmantelfläche des Führungsschafts (13) und als im Schiebeelement (14) radial zu der Rastausnehmung (41) verlaufende federbelastete Kugel (39) ausgebildet ist.

10. Endoskopische Schneideinrichtung nach Patentanspruch 1, **dadurch gekennzeichnet, dass** die Schneideinrichtung (1) in einem hohlzylindrischen Sondenkörper (6) einer Sonde (3) für eine perkutane endoskopische Gastrostomie anordenbar ist, deren Sondenkörper (6) gemeinsam mit einer Halteplatte (7) zur Anlage an einer Magenwand (4) ausgebildet und nach einer Überwucherung durch die Magenmukosa (9) mittels radialer Schnitte, die mit der Schneideinrichtung (1) durchgeführt werden können, aus dieser herauslösbar ist, und dass ein distales Ende des Sondenkörpers (6), das einen axialen Überstand über die Halteplatte (7) aufweist, hinsichtlich seiner axialen und radialen Abmessungen an die Lage des Schneiddrahtes (16) und die Lage eines als Spitze (20) ausgebildeten distalen Endes des Tubus (10) angepasst ist, die diese während eines Schneidvorganges einnehmen können.

## Claims

1. Endoscopic cutting device (1) for use when cutting free a probe (3) which is provided with a retaining plate (7), is arranged in a stomach wall (4) in the context of a percutaneous endoscopic gastrostomy and is overgrown with the gastric mucosa (9), wherein the cutting device (1) has a cutting element provided at a distal end of a tube (10) and, in order to adjust the cutting element, an actuation force of a proximally provided actuation element (11) can be transmitted to said cutting element via a pull cable (24) guided in a lumen (25) of the tube (10), wherein the cutting device (1) has an electrically heatable cutting wire (16) which is connected to the pull cable (24) and is fixed at a first distance to the distal end on the tube (10) and extends from this outside the tube (10) via a distal portion and is guided into the lumen (25) through a first opening (29) provided in the tube (10) at a second, longer distance to the distal end of the tube (10), such that, when the pull cable (24) is tensioned, the portion of the tube (10) deforms in an arc shape and the cutting wire (16) forms a first diathermy blade (17) extending like a chord between the first opening (29) and its fixing position on the tube (10), wherein, with the first distance to the end of the tip (20), a second opening (34) is formed in the tube (10), through which opening (34) the cutting wire (16) enters the lumen (25), and wherein the tube (10), at least in the region of its portion lying between the two openings (29 and 34), is made of a flexible, elastically deformable plastic, **characterized in that**, in order to form a tip (20), the distal end of the tube (10) is bevelled at an angle to its transverse plane directed towards the first diathermy blade (17), **in that** the cutting wire (16) extends in the lumen (25) as far as the tip (20), and **in that**, in order to form a second diathermy blade (36), the cutting wire (16) is guided on the outside around the tip (20) and, extending along an outer circumferential surface (31) of the tube (10), enters the tube (10) through the second opening (34).

2. Endoscopic cutting device according to Claim 1, **characterized in that** a distal end portion of the cutting wire (16) within the lumen (25) extends over at least 50% of the region lying between the two openings (29 and 34).

3. Endoscopic cutting device according to Claim 2, **characterized in that** the distal end portion within the lumen (25) extends as far as the first opening (29).

4. Endoscopic cutting device according to Claim 1, **characterized in that** the cutting wire (16), guided on the outside around the tip (20), extending along the outer circumferential surface (31) of the tube (10) and entering the lumen (25) of the tube (10) through the second opening (34), is bent through approximately 180° at its distal end and is fixed with this end to an inner circumferential surface of the lumen (25).

5. Endoscopic cutting device according to Claim 2, **characterized in that**, adjacent to the openings (29 and 34) and outside the region lying between the openings (29 and 34), PEEK sleeves (28 and 35) are inserted into the tube (10), preferably into the lumen (25).

6. Endoscopic cutting device according to Claim 5, **characterized in that** a first PEEK sleeve (35) extends within the lumen (25) from the tip (20) as far as the second opening (34).

7. Endoscopic cutting device according to Claim 5, **characterized in that** a PEEK hose (27), extending within the lumen (25) formed in the tube (10), is fixed with its proximal end in a receptacle (33) of a guide shaft (13) of the actuation element (11) and bears distally on a front face of a second PEEK sleeve (28) that extends as far as the first opening (29).

8. Endoscopic cutting device according to Claim 1, **characterized in that** the actuation element (11) has a guide shaft (13), connected at least indirectly to the tube (10), and a pushing element (14), engaging on the pulling cable (24), and an element (37) for triggering a haptic signal, which is triggered when the cutting device (1) has reached one of its cutting positions, is provided between guide shaft (13) and pushing element (14).

9. Endoscopic cutting device according to Claim 8, **characterized in that** the element (37) for triggering the haptic signal is configured as a latch recess (41) on an outer circumferential surface of the guide shaft (13) and as a springloaded ball (39) extending in the pushing element (14) radially with respect to the latch recess (41).

10. Endoscopic cutting device according to Claim 1, **characterized in that** the cutting device (1) can be arranged in a hollow cylindrical probe body (6) of a probe (3) for percutaneous endoscopic gastrostomy, which probe body (6) is configured together with a retaining plate (7) to bear on a stomach wall (4), and, after being overgrown with the gastric mucosa (9), is removable from the stomach wall by means of radial cuts that can be made with the cutting device (1), and **in that** a distal end of the probe body (6), having an axial protrusion beyond the retaining plate (7), is adapted in terms of its axial and radial dimensions to the position of the cutting wire (16) and to the position of a distal end of the tube (10) configured as a tip (20), which positions these can adopt during a cutting procedure.

## Revendications

1. Dispositif de coupe endoscopique (1) destiné à une utilisation lors du dégagement d'une sonde (3) munie d'une plaque de maintien (7), disposée dans une paroi d'estomac (4) dans le cadre d'une gastrostomie endoscopique percutanée et envahie par la muqueuse gastrique (9), dans lequel le dispositif de coupe (1) présente un élément de coupe prévu à une extrémité distale d'un tube (10), auquel une force d'actionnement d'un élément d'actionnement prévu en position proximale peut être transmise en vue de son réglage par l'intermédiaire d'un câble de traction (24), qui est guidé dans une lumière (25) du tube (10), dans lequel le dispositif de coupe (1) présente un fil de coupe (16) pouvant être chauffé électriquement relié au câble de traction (24), qui est fixé au tube (10) à une première distance de l'extrémité distale, s'étend à partir de celle-ci à l'extérieur du tube (10) sur une partie distale et est guidé dans la lumière (25) par une première ouverture (29) prévue dans le tube (10), qui est prévue à une deuxième distance plus grande de l'extrémité distale du tube (10), de telle manière que lors de la tension du câble de traction (24) la partie du tube (10) se déforme en forme d'arc et que le fil de coupe (16) forme un premier couteau de diathermie (17) s'étendant à la manière d'une corde entre la première ouverture (29) et sa fixation au tube (10), dans lequel une deuxième ouverture (34) est formée dans le tube (10) avec la première distance de l'extrémité de la pointe (20), par laquelle le fil de coupe (16) pénètre dans la lumière (25) et dans lequel le tube (10) se compose d'une matière plastique souple élastiquement déformable au moins dans la région de sa partie située entre les deux ouvertures (29 et 34),
**caractérisé en ce que** l'extrémité distale du tube (10) est biseautée sous un angle par rapport à son plan transversal pour la formation d'une pointe (20) qui est tournée vers le premier couteau de diathermie (17), **en ce que** le fil de coupe (16) s'étend dans la lumière (25) jusqu'la pointe (20) et **en ce que** le fil de coupe (16) est mené extérieurement autour de la pointe (20) pour la formation d'un deuxième couteau de diathermie (36), et pénètre dans le tube (10) par la deuxième ouverture (34) en s'étendant le long d'une surface latérale extérieure (31) du tube (10).

2. Dispositif de coupe endoscopique selon la revendication 1, **caractérisé en ce qu'**une partie d'extrémité distale du fil de coupe (16) s'étend à l'intérieur de la lumière (25) sur au moins 50 % de la région située entre les deux ouvertures (29 et 34).

3. Dispositif de coupe endoscopique selon la revendication 2, **caractérisé en ce que** la partie d'extrémité distale s'étend à l'intérieur de la lumière (25) jusqu'à la première ouverture (29).

4. Dispositif de coupe endoscopique selon la revendication 1, **caractérisé en ce que** le fil de coupe (16) mené extérieurement autour de la pointe (20) et s'étendant le long de la surface latérale extérieure (31) du tube (10), qui pénètre dans la lumière (25) du tube (10) par la deuxième ouverture (34), est replié d'environ 180° à son extrémité distale et est fixé par celle-ci à une surface latérale intérieure de la lumière (25).

5. Dispositif de coupe endoscopique selon la revendication 2, **caractérisé en ce que** des douilles en PEEK (28 et 35) sont insérées dans le tube (10), de préférence dans la lumière (25), à proximité des ouvertures (29 et 34) et à l'extérieur de la région située entre les ouvertures (29 et 34).

6. Dispositif de coupe endoscopique selon la revendication 5, **caractérisé en ce qu'**une première douille en PEEK (35) s'étend à l'intérieur de la lumière (25) depuis la pointe (20) jusqu'à la deuxième ouverture (34).

7. Dispositif de coupe endoscopique selon la revendication 5, **caractérisé en ce qu'**un tuyau souple en PEEK (27) s'étendant à l'intérieur de la lumière (25) formée dans le tube (10) est fixé avec son extrémité proximale dans un logement (33) d'une tige de guidage (13) de l'élément d'actionnement (11) et s'applique en position distale sur un côté frontal d'une deuxième douille en PEEK (28), qui s'étend jusqu'à la première ouverture (29).

8. Dispositif de coupe endoscopique selon la revendication 1, **caractérisé en ce que** l'élément d'actionnement (11) présente une tige de guidage (13) assemblée au moins indirectement au tube (10) et un élément coulissant (14) attaché au câble de traction (24), entre lesquels il est prévu un élément (37) pour déclencher un signal haptique, qui est déclenché lorsque le dispositif de coupe (1) a atteint une de ses positions de coupe.

9. Dispositif de coupe endoscopique selon la revendication 8, **caractérisé en ce que** l'élément (37) pour déclencher le signal haptique est formé par un évidement d'encliquetage (41) sur une surface latérale extérieure de la tige de guidage (13) et une bille à ressort (39) s'étendant radialement par rapport à l'évidement d'encliquetage (41) dans l'élément coulissant (14).

10. Dispositif de coupe endoscopique selon la revendication 1, **caractérisé en ce que** le dispositif de coupe (1) peut être disposé dans un corps de sonde cylindrique creux (6) d'une sonde (3) pour une gastrostomie endoscopique percutanée, dont le corps de sonde (6) est réalisé ensemble avec une plaque de maintien (7) destinée à être appliquée sur une paroi d'estomac (4) et peut, après un envahissement par la muqueuse gastrique (9), être extrait de celle-ci au moyen de coupes radiales, qui peuvent être effectuées avec le dispositif de coupe (1), et **en ce qu'**une extrémité distale du corps de sonde (6), qui présente un dépassement axial au-delà de la plaque de maintien (7), est adaptée en ce qui concerne ses dimensions axiales et radiales à la position du fil de coupe (16) et à la position d'une extrémité distale du tube (10) réalisée en forme de pointe (20), que ceux-ci peuvent prendre pendant une opération de coupe.
